# EUROPEAN PATENT SPECIFICATION

(11) **EP 3 479 804 B1**
(45) Date of publication and mention of the grant of the patent: **26.08.2020**
(21) Application number: 17823706.1
(22) Date of filing: 22.06.2017
(51) Int. Cl.: A61F 13/02

(54) **ADHESIVE FASCIAL BANDAGE**
HAFTENDE FASZIENBINDE
BANDE DE FASCIATHÉRAPIE ADHÉSIVE

(30) Priority: 05.07.2016 ES 201630912
(43) Date of publication of application: 08.05.2019
(73) Proprietor: Selva Sarzo, Francisco, 46022 Valencia (ES)
(72) Inventor: Selva Sarzo, Francisco, 46022 Valencia (ES)
(74) Representative: Clarke Modet & Co.
(86) International application number: PCT/ES2017/070455
(87) International publication number: WO 2018/007662

(56) References cited:
- WO-A1-01/97732
- WO-A1-2013/011384
- ES-A2- 2 558 021
- ES-A2- 2 558 021
- ES-T3- 2 318 758
- US-A- 4 207 885

## Description

### Field of the invention

The present invention belongs to the technical field of the industry dedicated to the manufacture of tapes, and more particularly, for bandages in the field of physical therapy, rehabilitation and medicine.

The invention particularly relates to an adhesive fascial tape with a composition of yarns and fabrics in its structure which offer greater connection and stabilization of forces between fibers during application, improving and facilitating handleability when placing it, providing optimal, limited and reproducible elongation which does not restrict body movement.

### Background of the invention

In recent years, tapes for treating musculoskeletal system disorders and injuries have been used without taking into consideration the effect they have on different fascial levels.

In order to comprehend the effects of the bandage, it is very important to highlight how closely interconnected the skin and fasciae are, and how closely interconnected the fasciae are with all the tissues in the body, mainly in the musculoskeletal system, affecting the working thereof. Therefore, they intervene in visceral, nervous or circulatory motility and mobility, affecting the musculoskeletal system, which is where more pain occurs, although the source of the injury is not always in said system.

Fasciae are an uninterrupted series of resistant connective tissues which are, however, retractable with inervation and vascularization, and they can be found from the head down to the feet and from the exterior to the interior of the body, a continuous tissue enveloping the entire body and all the structures contained therein. Taking into account that the deepest part of the skin, the hypodermis, is attached to the superficial fascia, the stimuli and mechanical effects generated in the skin are transmitted to superficial fasciae and from these to deep fasciae.

Anatomical and histological studies have shown that fasciae are found in all regions of the body and which, in turn, are formed by different layers, having an oblique, transverse or circular direction, so the general aspect of fasciae is one of a spiral.

Fasciae perform many functions in the body, among which those that apply for the case of the support function, hemodynamic function and communication function stand out, with the communication function being what allows all the connective tissues such as subcutaneous tissue, muscles, ligaments, tendons and bones to be interconnected by fasciae and to work synergistically. This aspect also explains how stimuli applied by the bandage to the skin are also transmitted to deep tissues, improving vascularization and inervation of the bandaged area.

Therefore, there is a need for tapes which help to normalize fascial problems due to either an excess or deficiency of tension which may even restrict motility, in addition to analytical and global mobility of the body, causing pain in different locations since a deficiency of tension in one area causes an excess thereof in another area, and vice versa, causing pain.

Kinesiology tape or Kinesio Taping, which consists of a system of cotton tapes incorporating a layer of adhesive with ripples which confer tack thereto but allow the skin to transpire, which are applied on the skin following a specialized technique, is already known in the state of the art. It is a type of elastic cotton tape.

Patent document ES2558021A2, which describes an elastic adhesive tape with an openwork structure with a satin or twill weave, with weft yarns being of cotton (100%) and warp yarns being a blend of cotton and elastomer (95% and 5% respectively), is also known.

There are other tapes, such as rigid tape (athletic tape or strappal), which is a non-elastic, hypoallergenic, adhesive tape that can be torn by hand in both directions, particularly applied for functional bandages. It contains a taffeta-type fabric, which confers a high tensile strength thereto.

However, there are no tapes that are truly functional since their technical characteristics do not allow them to be used in multiple activities in which wide ranges of movement are required, such as the shoulder, elbow or knee joints, and where it is necessary to produce a specific and reproducible tension at an elastic point far from the neutral starting position of the bandage since its main component is cotton.

Likewise, when the area to be bandaged has little fascial mobility (lumbar region, lateral regions of the ankle), there is a tendency to create a great deal of tension when placing the bandage since it is neither specific nor reproducible, producing an excessive stimulus in said region, injuring the taped skin, compressing local inervation and vascularization, in addition to creating fascial compensations in other regions of the body.

When cotton fibers are wet, they readily absorb and maintain liquids such as water or sweat, increasing their weight. Once the fabric is wet, ventilation drops and will deteriorate, taking a long time to dry, and this may be detrimental to the performance of the glue. Therefore, the skin of the patient will always be wet, and accordingly, the capacity to be kept fresh will decrease exponentially, as will the adhesion of the bandage, casting a doubt on the therapeutic success of the bandage. Furthermore, this feeling of heaviness, moisture and the possible bad odor that it may give off is unpleasant for the user.

Therefore, if the cotton tape gets wet, it absorbs the liquid, increasing the weight of the tape, and the drying time increases, with said drying being slow. Transpiration decreases, and the capacity to keep the skin fresh since, by being in contact with sweat, the fibers become wet and the body loses heat through conduction. All this affects skin quality, where small wounds, irritations or itching may occur, in addition to affecting the properties of the tape and the glue, reducing adhesion.

It was therefore desirable to provide a tape with a suitable structure and suitable materials which will provide greater connection and stabilization of forces between fibers during application, improving and facilitating handleability when placing it, providing optimal, limited, and reproducible elongation which does not restrict body movement, preventing the drawbacks existing in the previous tapes of the state of the art.

### Description of the invention

The present invention solves the problems the problems existing in the state of the art relating to adhesive tapes by means of the combination of its structure and materials, to improve the effects they produce and comfort.

The tape has a composition of yarns and fabrics in its structure which, unlike existing tapes, offers greater connection and stabilization of forces between fibers during application, improving and facilitating the handleability when placing it, being maintained during movement. The tape is designed to help prevent irritations, eczemas, edemas or Morel-Lavallee effusion caused by the high tension produced by the bandage and the limited or full movements of the body depending on the area to be bandaged.

The structure of the fabric of the fascial tape is an openwork structure, with a plain, twill or satin weave of 3, where a satin or twill weave of up to 10 can be used, with a density of warp yarns of 1 to 40 picks/cm and of weft yarns of 1 to 35 yarns/cm. Said weave can be broken or crossed, in which each yarn takes two consecutive picks and leaves the next two picks free, having the same effects on both faces.

By increasing the structure used in the earlier technique, making a structure of 10, there is less friction between the yarns, therefore said yarns are less gripped together by friction since loops (float stitches on the surface of the fabric) are created which help the tape to have greater elastic strength.

On the other hand, if only the structure is taken into account without taking the yarns used into account, by increasing the length of the loops, elastic strength is increased, but its mechanical strength and gripping, which are indispensable for correct and controlled treatment, are reduced.

Therefore, another one of the fundamental elements in the invention are the yarns forming it, which yarns can control the mechanical strength and gripping by clustering the yarns in plies, cabled or as cores. The strength of the yarn can be even further defined by twisting different yarn fibers with the same structure or different structures.

Based on the densities of said yarns and picks, the grammage of the tape is calculated, being defined, together with the composition thereof, as the correct tenacity, maximum strength, total work and elongation necessary for the tape to correctly carry out its function without damaging the skin on which it is adhered or cause fascial decompensations that may cause contractures or limitations of mobility locally or in other parts of the body.

On the other hand, depending on the location of the skin on which the bandage will be placed, its elasticity and sensitivity will vary. Therefore, in order for the stimulation from the skin to have a proper influence, the bandage must respect the characteristics and properties of the skin of each segment of the body. The flat fabric of our tape is based on being non-elastic to being able to be elastic in the longitudinal, transverse and tangential directions, maintaining a high stability so as to respect the differences of cutaneous areas. Said elasticity will be determined by the combination of weft and warp of different fibers.

With respect to the yarns used, they will be filament yarns, selected from smooth and textured monofilament yarns, multifilament yarns, or combinations thereof, depending on the desired features. Therefore, to create suitable tapes for sports with restricted or full ranges of movement or for the treatment of specific pathologies, said yarns must be combined.

Furthermore, in order for the tape, which is intended for an intense use, to be rip resistant without limiting movement and without being detrimental to the skin, a square of a ripstop lightweight fabric is integrated, which provides resistance, durability and protection against ripping.

The tape is formed by natural fibers, by a combination of natural fibers with synthetic fibers or only by synthetic fibers, where metal fibers can additionally be used since they are yarns that cause few problems if they are used in their indicated manner.

In addition, said fibers can be magnetized in order to influence the magnetic fields of the body, influencing the regulation of body pH.

In summary, the structure of the yarns and fabrics of the fascial tape provides optimal, limited and controlled elongation which does not restrict body movement but controls the tension produced by the excessive elongation occurring in other tapes since it is designed to not contribute to developing eczema, Morel-Lavallee effusion or Morel-Lavallee extravasation. Where there is a sliding of the skin which exceeds its physiological limits of movement over the fascia, it causes the tearing and separation or avulsion of the skin and subcutaneous cell tissue of muscle fascia (degloving) affecting the proper working of the lymphatic system, creating an accumulation of lymph under the skin and being able to cause skin necrosis or serious infections.

By combining the high thermal capacity properties in the event of convection mechanisms convection *"Windchill"* or perceived temperature, being a good heat conductor, and pleasant feel and comfort of the natural fibers, with the properties of the artificial fibers, they absorb less water, dry faster, transpire and resist traction better, offering less friction and wear.

To repel water from the fabric without seeping through, polytetrafluoroethylene (PTFE) membrane or a thin or a thin layer of silicone oil which helps to waterproof the tape, limiting the flow of liquid which the tape can absorb. The outer part will be waterproof and the inner part will be thermal.

In a particular manner, spaces or lines without adhesive are created in the adhesive layer so that the tape can transpire, said spaces or lines creating a symmetric repetitive sinuous pattern with an angle of 45° with respect to the weft. Its adhesive ends are divided into two equal parts.

The spaces into which the adhesive areas are divided have an angle of 45°, equal to the angle of the spiral-shaped fascial fibers of the body, so the tape will react in a manner that is very similar to the fasciae when movement exists. These spaces form a pattern which allows ventilating and creating a quick and effective outlet for the sweat and/or water that are wetting the tape. They thereby help to eliminate the moisture that may be stored between the skin and the tape, favoring a drier, more hygienic and longer-lasting application.

On the other hand, with respect to elasticity, if the tape contains elastomer in warp, the tape will have longitudinal elasticity. If an elastomer is additionally added in the weft, longitudinal, transverse or tangential elasticity is obtained, improving movement and adhesion on the skin. The tape as a whole is hypoallergenic.

The drawbacks of the tapes of the state of the art are thereby overcome, providing a fascial tape which helps to improve problems with both superficial fasciae and deep fasciae, therefore it helps to alleviate pain and problems which are reflected or occur in the musculoskeletal system.

### Brief description of the drawings

To help understand the invention, an embodiment of the invention will be described below by way of non-limiting illustration in reference to a series of Figures.
Figures 1 and 2 are front and profile views, respectively, of a structure of a fabric (1) of weft yarns (2) and warp yarns (3) with an openwork structure with a twill or satin weave of 10.
Figure 3 is a side view of the tape, with the fabric (1) and the adhesive layer (4) being distinguished.
Figure 4 is a view of the cross section taken along line A-A of Figure 3.
Figure 5 is the pattern of the shape of the adhesive layer (4), where the areas without adhesive (5) and the areas with adhesive (6) are distinguished.

### Detailed description of the invention

The object of the present invention is an adhesive fascial tape with a suitable structure and suitable materials which will provide greater connection and stabilization of forces between fibers during application, improving and facilitating handleability when placing it.

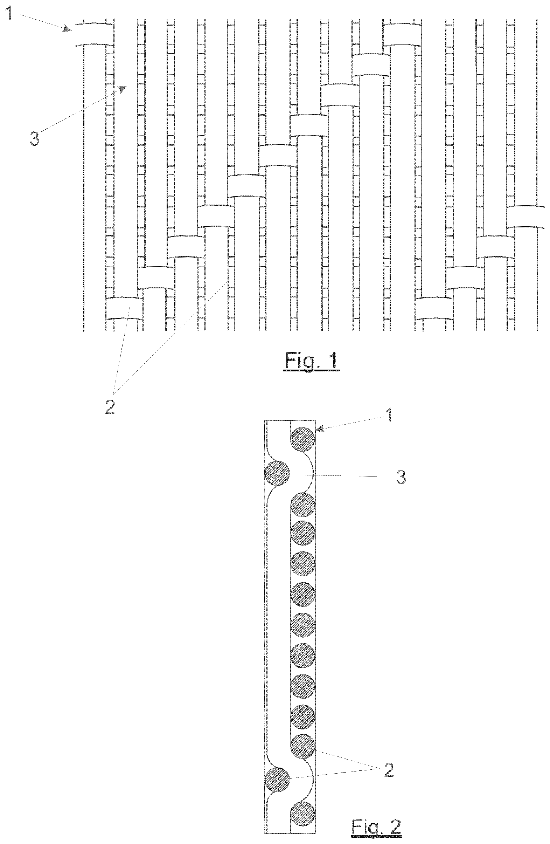

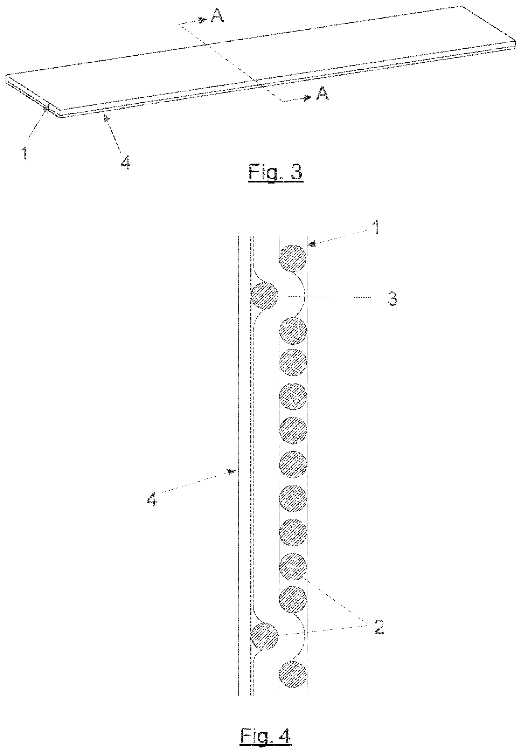

Thus, as can be seen in said Figures 1-3, the fabric (1) of the tape is made up of filament yarns the composition of which is consists of cotton and/or nylon and polyester fibers which are twisted into plied yarns or cabled yarns, said fabric being formed by a structure of weft yarns (2) and warp yarns (3).

It should be pointed out that the weft yarns (2) of the fabric of the tape have a density of 1 yarn/cm up to 35 yarns/cm and the warp yarns (3) of 1 pick/cm up to 40 picks/cm.

The distribution of the materials in twisted yarns facilitates the control of the elongation of the structure of a plain, twill or satin weave of 3 and up to 10 by clarifying the correct tension of the tape required for the elongation of the skin of each body segment without being detrimental to it. Said weave can be broken or crossed, in which each yarn takes two consecutive picks and leaves the next two picks free, having the same effects on both faces.

As the tape a mixed composition of natural and synthetic fibers, preferably cotton, nylon and polyester fibers, friction drops so the tape is suitable for being applied in regions of the body with less elongation of the skin, such as the lumbar region.

The tape is preferably elastic without requiring elastomer since the fibers thereof are made up of artificial elastic and cotton fibers. If greater elasticity is desired, plied or cabled yarns including elastomer can be created.

With respect to the yarns forming the tape, they are twisted together forming plied yarns, preferably having 2, 3 and 4 plies, where the yarns are twisted together to created cabled yarn, preferably having 4, 6 or 9 cables. Furthermore, yarns with a core located in the direction in which more elongation is to be created are used.

The yarns are filament yarns, selected from smooth and textured monofilament yarns, multifilament yarns, or combinations thereof. If more strength, uniformity and not having flexibility are desired, monofilament yarns will be used. If the most essential requirement is strength, smooth multifilament yarn is used. If it is necessary to provide the fiber with more coverage and extendibility, a textured filament yarn is used.

It should be pointed out that the tape has the following mechanical properties: tenacity between 0.05 kg/mm and 0.99 kg/mm, maximum force between 5 kg and 28 kg and total work between 0.05 kg/mm and 0.99 kg/mm. It further comprises a grammage of between 100 g/m² and 300 g/m².

Said grammage is optimal and necessary so that the energy absorbed by the material with the subsequent deformations it acquires during elongation before tearing (tenacity) and the maximum strength thereof are appropriate. The total work produced controls and normalizes elongation thereof, which allows the tape to remain intact and functional in any situation, even if it is in an advanced stage of elastic elongation.

Finally, it must be pointed out that the lower layer (4) of adhesive is preferably a thin layer of acrylic synthetic filament yarn, preferably without latex, with a high adhesive power on the skin and on the material of the yarns.

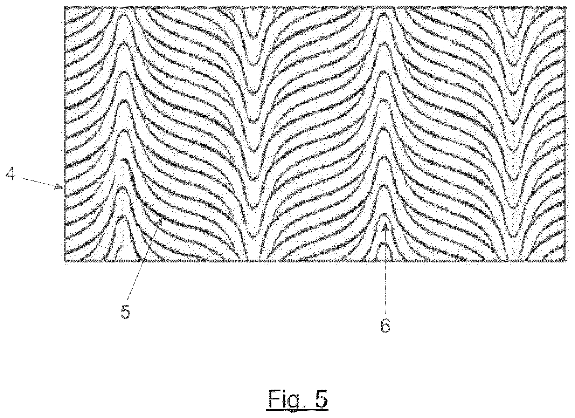

An infinitely repeating pattern forming the spaces or grooves without adhesive (5) incorporated for better evacuation of sweat or any liquid that may accumulate between the skin and the tape can be seen in Figure 5. The rest of the surface is covered with adhesive (6). Said spaces allow preventing malformations of the tape on the skin after application that may pinch or hurt the skin of the user, since the winding shape of the spaces or lines without adhesive (5) have an angle of 45°, preferably the direction of the weft, which further facilitates removal of the tape.

The tape is adhered on a paper with a previously established elongation. Said elongation keeps the fibers slightly tensed, protecting the mechanical properties and characteristics thereof. If the fibers are not adhered on the paper somewhat tensed, they may lose their correct functionality.

Having clearly described the invention, it must be stated that the particular embodiments described above are susceptible to modifications in detail provided that they do not alter the fundamental principle and essence of the invention.

## Claims

1. Adhesive fascial tape, **characterized in that** it comprises a flat fabric with weft and warp (1), forming an openwork structure with a plain, twill or satin weave, broken or crossed both in weft and in warp with a lower layer (4) of adhesive, wherein the yarn forming the fabric are filament yarns clustered in plies, cabled or as cores comprising natural and/or synthetic fibers,
wherein the yarns of the flat fabric with weft and warp (1) form an openwork structure with a plain, twill or satin weave of at least 3 and up to 10, the weft yarns (2) of the structure of the tape have a density of 1 up to 35 picks/cm and the warp yarns (3) of the structure of the tape have a density of 1 up to 40 yarns/cm, and wherein the natural and/or synthetic fibers forming the fabric are chosen from the group comprising cotton, nylon, polyester, polypropylene and elastane, or combinations thereof.

2. Adhesive fascial tape according to claim 1, **characterized in that** the filament yarns are selected from smooth or textured monofilament yarns, multifilament yarns and/or combinations thereof.

3. Adhesive fascial tape according to claims 1 to 2, **characterized in that** the fabric contains a square of ripstop lightweight fabric.

4. Adhesive fascial tape according to claims 1 to 3, **characterized in that** it has a grammage of between 100 g/m² and 300 g/m².

5. Adhesive fascial tape according to claims 1 to 4, **characterized in that** it comprises magnetized metal fibers.

6. Adhesive fascial tape according to claims 1 to 5, **characterized in that** it comprises magnetized fibers.

7. Adhesive fascial tape according to claims 1 to 6, **characterized in that** the lower layer (4) comprises spaces or lines without adhesive (5), said spaces or lines creating a symmetric repetitive sinuous pattern with an angle of 45° with respect to the weft.

8. Adhesive fascial tape according to claims 1 to 7, **characterized in that** it comprises an upper layer containing a polytetrafluoroethylene (PTFE) membrane or a thin layer of silicone oil which helps to waterproof the tape.

9. Adhesive fascial tape according to claims 1 to 8, **characterized in that** it comprises a protective layer that can be removed before use to protect its characteristics and properties.

## Patentansprüche

1. Haftende Faszienbinde, **dadurch gekennzeichnet, dass** sie ein flaches Gewebe mit Schuss und Kette (1) umfasst, das eine durchbrochene Struktur mit einer Leinen-, Körper- oder Satinbindung bildet, die sowohl in dem Schuss als auch der Kette mit einer unteren Schicht (4) von Klebstoff überkreuzt oder gebrochen ist, wobei das Garn, das das Gewebe bildet, Filamentgarne sind, die in Lagen, verkabelt oder als Kerne, die Naturfasern und/oder synthetische Fasern umfassen, gruppiert sind,
wobei die Garne des flachen Gewebes mit Schuss und Kette (1) eine durchbrochene Struktur mit einer Leinen-, Körper- oder Satinbildung von mindestens 3 und bis 10 bilden, wobei die Schussgarne (2) der Struktur der Binde eine Dichte von 1 bis 35 Picks/cm aufweisen und die Kettgarne (3) der Struktur der Binde eine Dichte von 1 bis 40 Garne/cm aufweisen, und wobei die Naturfasern und/oder synthetischen Fasern, die das Gewebe bilden, aus der Gruppe ausgewählt sind, die Baumwolle, Nylon, Polyester, Polypropylen und Elastan oder Kombinationen davon umfasst.

2. Haftende Faszienbinde nach Anspruch 1, **dadurch gekennzeichnet, dass** die Filamentgarne aus glatten oder strukturierten Monofilamentgarnen, Multifilamentgarnen und/oder Kombinationen davon ausgewählt sind.

3. Haftende Faszienbinde nach den Ansprüchen 1 bis 2, **dadurch gekennzeichnet, dass** das Gewebe ein Quadrat aus leichtem Ripstop-Gewebe enthält.

4. Haftende Faszienbinde nach den Ansprüchen 1 bis 3, **dadurch gekennzeichnet, dass** sie ein Flächengewicht zwischen 100 g/m² und 300 g/m² aufweist.

5. Haftende Faszienbinde nach den Ansprüchen 1 bis 4, **dadurch gekennzeichnet, dass** sie magnetisierte Metallfasern umfasst.

6. Haftende Faszienbinde nach den Ansprüchen 1 bis 5, **dadurch gekennzeichnet, dass** sie magnetisierte Fasern umfasst.

7. Haftende Faszienbinde nach den Ansprüchen 1 bis 6, **dadurch gekennzeichnet, dass** die untere Schicht (4) Räume oder Linien ohne Klebstoff (5) umfasst, wobei die Räume oder Linien ein symmetrisches, sich wiederholendes sinusförmiges Muster mit einem Winkel von 45° bezüglich des Schusses erzeugen.

8. Haftende Faszienbinde nach den Ansprüchen 1 bis 7, **dadurch gekennzeichnet, dass** sie eine obere Schicht umfasst, die eine Polytetrafluorethylen(PTFE)-membran oder eine dünne Schicht aus Silikonöl enthält, welche dabei hilft, die Binde wasserdicht zu machen.

9. Haftende Faszienbinde nach den Ansprüchen 1 bis 8, **dadurch gekennzeichnet, dass** sie eine Schutzschicht umfasst, die vor der Nutzung entfernt werden kann, um ihre Merkmale und Eigenschaften zu schützen.

## Revendications

1. Bande de fasciathérapie adhésive, **caractérisée en ce qu'**elle comprend un tissu plat avec trame et chaîne (1), formant une structure ajourée avec une armure toile, sergé ou satin, rompue ou croisée à la fois en trame et en chaîne avec une couche inférieure (4) d'adhésif, dans lequel les fils formant le tissu sont des fils de filaments regroupés en nappes, câblés ou sous forme d'âmes comprenant des fibres naturelles et/ou synthétiques,
dans lequel les fils du tissu plat avec trame et chaîne (1) forment une structure ajourée avec une armure toile, sergé ou satin d'au moins 3 et jusqu'à 10, les fils de trame (2) de la structure de la bande ont une densité de 1 jusqu'à 35 fils par cm et les fils de chaîne (3) de la structure de la bande ont une densité de 1 jusqu'à 40 fils par cm, et dans lequel les fibres naturelles et/ou synthétiques formant le tissu sont choisies dans le groupe comprenant le coton, le nylon, le polyester, le polypropylène et l'élasthanne, ou des combinaisons de ceux-ci.

2. Bande de fasciathérapie adhésive selon la revendication 1, **caractérisée en ce que** les fils de filaments sont choisis parmi des fils monofilaments lisses ou texturés, des fils multifilaments et/ou des combinaisons de ceux-ci.

3. Bande de fasciathérapie adhésive selon les revendications 1 à 2, **caractérisée en ce que** le tissu contient un carré de tissu léger ripstop.

4. Bande de fasciathérapie adhésive selon les revendications 1 à 3, **caractérisée en ce qu'**elle a un grammage compris entre 100 g/m² et 300 g/m².

5. Bande de fasciathérapie adhésive selon les revendications 1 à 4, **caractérisée en ce qu'**elle comprend des fibres métalliques magnétisées.

6. Bande de fasciathérapie adhésive selon les revendications 1 à 5, **caractérisée en ce qu'**elle comprend des fibres magnétisées.

7. Bande de fasciathérapie adhésive selon les revendications 1 à 6, **caractérisée en ce que** la couche inférieure (4) comprend des espaces ou des lignes sans adhésif (5), lesdits espaces ou lignes créant un motif sinueux répétitif symétrique avec un angle de 45° par rapport à la trame.

8. Bande de fasciathérapie adhésive selon les revendications 1 à 7, **caractérisée en ce qu'**elle comprend une couche supérieure contenant une membrane de polytétrafluoroéthylène (PTFE) ou une fine couche d'huile de silicone qui aide à imperméabiliser la bande.

9. Bande de fasciathérapie adhésive selon les revendications 1 à 8, **caractérisée en ce qu'**elle comprend une couche de protection qui peut être enlevée avant l'utilisation pour protéger ses caractéristiques et ses propriétés.
